# EUROPÄISCHE PATENTSCHRIFT

(11) **EP 0 827 944 B1**
(45) Veröffentlichungstag und Bekanntmachung des Hinweises auf die Patenterteilung: **28.11.2001**
(21) Anmeldenummer: 97115340.8
(22) Anmeldetag: 04.09.1997
(51) Int. Cl.: C07C 7/163, B01J 35/06

(54) **Verfahren zur Hydrierung**
Hydrogenation process
Procédé d'hydrogénation

(30) Priorität: 05.09.1996 DE 19636064
(43) Veröffentlichungstag der Anmeldung: 11.03.1998
(73) Patentinhaber: BASF AKTIENGESELLSCHAFT, 67056 Ludwigshafen (DE)
(72) Erfinder: Flick, Klemens, Dr., 76863 Herxheim (DE); Freire Erdbrügger, Christina, Dr., 67240 Bobenheim-Roxheim (DE); Bröcker, Franz Josef, Dr., 67061 Ludwigshafen (DE); Meyer, Gerald, Dr., 67071 Ludwigshafen (DE); Schwab, Ekkehard, Dr., 67434 Neustadt (DE); Herion, Christof, Dr., 68526 Ladenburg (DE)
(74) Vertreter: Isenbruck, Günter, Dr.

(56) Entgegenhaltungen:
- EP-A- 0 053 884
- EP-A- 0 412 415
- EP-A- 0 564 830
- DE-A- 2 710 277
- PATENT ABSTRACTS OF JAPAN vol. 013, no. 260 (C-607), 15.Juni 1989 & JP 01 061433 A (SHINDAIKIYOUWA SEKIYU KAGAKU KK), 8.März 1989,

## Beschreibung

Die Erfindung betrifft Verfahren zur Hydrierung von C₂₋₈-Alkinen und/oder C₄₋₈-Alkinenen und/oder C₄₋₈-Alkadienen in diese enthaltenden Fluiden. Alkine, wie Acetylene, und Diene sind aufgrund ihrer Neigung zur Polymerisation oder ihrer ausgeprägten Neigung zur Komplexbildung an Übergangsmetallen in vielen technischen Synthesen unerwünschte Stoffe. Sie beeinträchtigen die bei diesen Reaktionen verwendeten Katalysatoren zum Teil sehr stark. So stört z.B. das im C₂-Strom eines Steamcrackers enthaltene Acetylen die Polymerisation des Ethylens, so daß der Acetylengehalt im C₂-Strom sehr klein gehalten werden muß, vorzugsweise kleiner 1 ppm. Auch der C₃-Strom eines Steamcrackers, der neben Propylen auch 2 bis 3% Propadien (PD) und etwa die gleiche Menge Propin (Methylacetylen, MA) enthält, muß vor der Polymerisation zu Polypropylen gereinigt werden. Der typische Gehalt an mehrfach ungesättigten Kohlenwasserstoffen liegt dabei bei etwa 4 bis 6 Gew.-%. Eine Verringerung dieses Gehalts auf maximal 10 ppm sollte vorzugsweise erreicht werden. Auch der C₄-Strom eines Steamcrackers enthält bis zu 70 % an mehrfach ungesättigten Kohlenwasserstoffen. Diese bestehen hauptsächlich aus Butadien, Vinylacetylen und Ethylacetylen. Der Gesamtgehalt an mehrfach ungesättigten Kohlenwasserstoffen sollte auf unter 20 ppm, vorzugsweise maximal 10 ppm vermindert werden. Dies wird in der Praxis durch selektive Hydrierung der Kohlenwasserstoffströme an heterogenen Edelmetallkatalysatoren auf keramischen Trägern erreicht. Hierbei werden hohe Anforderungen an die verwendeten Hydrierkatalysatoren hinsichtlich ihrer Selektivität und Aktivität gestellt, denn es soll eine möglichst vollständige Hydrierung der mehrfach ungesättigten Kohlenwasserstoffe ohne Verlust an einfach ungesättigten Kohlenwasserstoffen, wie Ethylen, Propen oder Butenen erreicht werden.

In manchen Fällen sollen durch Selektivhydrierung eines Roh-C₄-Stroms aus einem Steamcracker, der etwa 40 bis 60 Gew.-% Butadien enthält, Butene in möglichst großer Ausbeute gebildet werden. Auch in diesem Fall werden industriell heterogene Edelmetallkatalysatoren auf keramischen Trägern eingesetzt. Für diese Einsatzzwecke wurden promotierte oder unpromotierte Edelmetallkatalysatoren auf keramischen Trägern meist mit Palladium als Aktivkomponente in einer Menge von 0,01 bis 1 Gew.-% verwendet.

In den bekannten Verfahren wird dem Reaktionsgemisch bei der Hydrierung von Acetylen oft Kohlenmonoxid beigemischt, um die Selektivität des Katalysators zu erhöhen. Der Nachteil dieser Verfahren besteht darin, daß die selektivitätssteigernde Wirkung des Kohlenmonoxids stark temperaturabhängig ist. Große Temperaturgradienten im Katalysatorbett haben daher eine Verschlechterung der Selektivität zur Folge. Außerdem begünstigen die höheren notwendigen Arbeitstemperaturen bei der Zudotierung von Kohlenmonoxid eine vermehrte Bildung von unerwünschten Polymeren (Grünöl).

Die bekannten Katalysatoren für die selektive Hydrierung von mehrfach ungesättigten Verbindungen werden im allgemeinen durch Tränken eines inerten Trägers mit einer wässrigen Lösung eines Palladiumsalzes, eines Gemisches eines Palladiumsalzen mit einem Promotersalz oder durch aufeinanderfolgende getrennte Tränkung mit wässrigen Lösungen der als Katalysator und/oder Promoter aktiven Substanzen, nachfolgender Trocknung und Calcinierung bei höheren Temperaturen hergestellt. Die meisten der verfügbaren Katalysatoren werden nach dem Einbau in den Reaktor mit Wasserstoff reduziert.

In der DE-A 2 107 568 ist ein Verfahren zur Reinigung von Kohlenwasserstoffen durch Selektivhydrierung beschrieben. Mehrfach ungesättigte Verbindungen wie Methylacetylen und Propadien werden in der Flüssigphase in zwei hintereinandergeschalteten Reaktionszonen hydriert. In der ersten Reaktionszone verdampft ein Teil der Flüssigkeit. Als Katalysator wird Pd auf Al₂O₃ verwendet.

In der EP-A-0 653 243 sind Trägerkatalysatoren beschrieben, die erhalten werden durch Lösen von Palladiumnitratlösung, gegebenenfalls zusammen mit Silbernitratlösung in einem Lösungsmittel, Versetzen der Lösung mit einem hochmolekularen Natriumpolyacrylat und Vermischen mit Aluminiumoxid als Träger. Die erhaltene Masse wird geformt, getrocknet und kalziniert. Der Katalysator wird zur selektiven Hydrierung von Methylacetylen und Propadien in einem C₃-Strom in der Flüssigphase verwendet.

In der EP-A-0 532 482 ist ein Verfahren zur selektiven Hydrierung von butadienreichen Roh-C₄-Schnitten beschrieben. Die selektive Hydrierung von Butadien zu Butenen erfolgt in der Flüssigphase an fest angeordneten Palladium-Trägerkatalysatoren. Die Hydrierung erfolgt in zwei hintereinandergeschaltenen Reaktionszonen.

In der DE-C-31 19 850 ist ein Verfahren zur selektiven Hydrierung von Butadien in einer C₄-Fraktion beschrieben. In der Flüssigphasenhydrierung wird 0,3 Gew.-% Palladium auf einem Aluminiumoxidträger in Form von Kugeln mit 2 mm Durchmesser eingesetzt.

In der EP-B-0 288 362 ist ein Verfahren zur Isomerisierung von 1-Buten zu 2-Butenen in einem C₄-Kohlenwasserstoffschnitt beschrieben, der Butadien und schwefelhaltige Verbindungen enthält. Der Kohlenwasserstoffschnitt wird über ein erstes Bett eines Katalysators geführt, der Palladium und Gold und/oder Platin enthält. Sodann wird der Strom über ein zweites Katalysatorbett geführt, das Palladium niedergeschlagen auf Aluminiumoxid oder auf Siliciumdioxid enthält.

In der US 4,260,840 ist ein Verfahren zur Reinigung eines 1-Buten enthaltenden Stroms beschrieben. Dabei wird Butadien selektiv zu Buten hydriert in einem C₄-Strom, der mindestens 30 Gew.-% 1-Buten enthält. Als Trägerkatalysator wird Pd/Cr auf Aluminiumoxid in einem gepackten Katalysatorbett verwendet.

In der US 5,475,173 ist ein Verfahren zur selektiven Hydrierung von 1,3-Butadien beschrieben. Der Katalysator enthält Palladium und Silber auf Al₂O₃ sowie ein Alkalimetallfluorid.

In der EP-B-0 064 301 ist ein Katalysator für die selektive Hydrierung von Acetylen beschrieben. Der Katalysator enthält 0,01 bis 0,025 Gew.-% Palladium und 2 bis 6 mal soviel Silber auf einem alpha-Al₂O₃-Träger mit einer Oberfläche von 3 bis 7 m²/g. Der Katalysator weist eine geringe CO-Empfindlichkeit und eine lange Standzeit auf.

Der EP-B-0 089 252 ist ein Katalysator zur selektiven Hydrierung von acetylenischen Kohlenwasserstoffen beschrieben. Der Katalysator weist 0,03 bis 1 Gew.-% Palladium und 0,003 bis 0,3 Gew,-% Gold auf einem Al₂O₃-Träger auf.

In der US 4,839,329 ist ein Verfahren zur Herstellung eines Palladiumkatalysators auf einem Titandioxidträger beschrieben. Der Palladiumgehalt beträgt 0,01 bis 0,2 Gew.-%. Der Katalysator ist geeignet zur selektiven Hydrierung von Acetylen zu Ethen.

In der DE-C 1 284 403 ist ein Verfahren zur Herstellung von Palladium-Schwermetall-Tonerde-Katalysatoren zur Entfernung von Acetylenen und Diolefinen aus vorwiegend Monoolefine enthaltenden Gasgemischen durch selektive Hydrierung beschrieben. Es wird Pd/Cr auf tonerdehaltigen Trägern zur Entfernung von Methylacetylen und Propadien eingesetzt.

In der DE-C 1 299 629 ist ein Verfahren zur Entfernung von Acetylenen aus vorwiegend Olefine enthaltenen Gasgemischen durch selektive Hydrierung beschrieben. Es wird ebenfalls ein Pd/Cr-Katalysator auf Tonerde zur Gasphasenhydrierung von Propadien und Methylacetylen verwendet.

Die bekannten Trägerkatalysatoren weisen die üblichen Nachteile von auf Oxiden geträgerten Katalysatoren auf. Sie zeigen Abrieb, sind empfindlich gegen mechanische Belastung bei Druckstößen bzw. dem Auftreten eines Druckverlusts über dem Katalysatorbett und sind bei Ein- oder Ausbau von frischem oder gebrauchtem Katalysator unangenehm zu handhaben.

In Catalysis Today, 24 (1995), Seiten 181-187 ist die Verwendung eines Monolithen aus α-Al₂O₃ mit einer Wandstärke von 0,2 mm und einer Zellendichte von 110 Zellen/cm² für die Selektivhydrierung von Acetylen im C₂-Stom ex Steamcracker in der Gas- uns Flüssigphase beschrieben.

JP-A-1061433 betrifft die Herstellung von Cycloolefinen durch Hydrierung von Cyclodienen. Dabei wird die Hydrierung an einem Katalysator durchgeführt, der aus einem dünnen Film von Palladium und Phosphor oder Bor auf einem Träger besteht. Der Katalysator mit dem dünnen Film der Aktivmaterialien wird durch Dampfabscheideverfahren wie Sputtern hergestellt. Der Träger wird jedoch nicht näher beschrieben.

DE-A-27 10 277 betrifft ein Verfahren zur Herstellung von wasserstoffdurchlässigen Membrankatalysatoren auf der Basis von Palladium oder dessen Legierungen zur Hydrierung ungesättigter organischer Verbindungen. Die Katalysatoren werden durch Aufbringen eines Polymers auf einen metallkeramischen Träger und Dampfabscheiden von Palladium auf das Polymer erhalten. Nach dem Dampfabscheiden wird die Polymerschicht vom Träger getrennt und als Membran eingesetzt. Mit dem Katalysator wird Cyclopentadien zu Cyclopenten hydriert.

EP-A-0 412 415 betrifft Palladiumkatalysatoren, die durch Bedampfen eines Trägermaterials mit metallischem Palladium erhalten werden, wobei die aufgedampfte Palladiumschicht mit einem metallischen Inhibitor durch Bedampfen und sodann thermisch behandelt wird. Der Träger kann dabei vorzugsweise aus metallischen Netzen, Folien oder Geweben ausgewählt sein. Das Trägermaterial kann die Form eines Monolithen aufweisen. Es wird eine selektive Hydrierung einer Dreifach- zu einer Doppelbindung in Gegenwart einer Hydroxylgruppe im Molekül (HDHL) in der Flüssigphase beschrieben, wobei die Hydroxylgruppe erhalten bleibt.

Ein Nachteil dieser keramischen Monolithe ist die fehlende Quervermischung in den einzelnen getrennten Kanälen und die Ausbildung von laminaren Strömungen bei langsamen Strömungsgeschwindigkeiten, was zu schlechteren Selektivitäten führt.

Eine Aufgabe der vorliegenden Erfindung ist die Bereitstellung von Katalysatoren zur selektiven Hydrierung mehrfach ungesättigter Kohlenwasserstoffe in Kohlenwasserstoffströmen.

Eine weitere Aufgabe der vorliegenden Erfindung ist die Bereitstellung eines Verfahrens zur Hydrierung von mehrfach ungesättigten Kohlenwasserstoffen, wobei die vorstehend beschriebenen Nachteile der bekannten Katalysatoren vermieden werden.

Gelöst werden die Aufgaben durch ein Verfahren zur selektiven Hydrierung von mehrfach ungesättigten C₂₋₆-Kohlenwasserstoffen, in diese enthaltenden C₂-, C₃-, C₄-, C₅- oder C₆-Strömen aus einem Steamcracker oder einem katalytischen Cracker zu den entsprechenden einfach ungesättigten Kohlenwasserstoffen durch Kontaktieren mit einer Katalysatorpackung in Gegenwart von freiem Wasserstoff, bei dem die Katalysatorpackung herstellbar ist durch Aufbringen mindestens einer als Katalysator und gegebenenfalls mindestens einen als Promotor aktiven Substanz auf Gewebe, Gestricke oder Folien als Trägermaterial.

Die erfindungsgemäß eingesetzten Katalysatoren weisen den nachstehend beschriebenen Aufbau auf.

### Trägermaterial

Als Trägermaterial für die erfindungsgemäß eingesetzten Katalysatoren können eine Vielzahl von Folien und Geweben, wie auch Gewirken, beispielsweise Gestricke eingesetzt werden. Es können erfindungsgemäß Gewebe mit unterschiedlicher Webart eingesetzt werden, wie glatte Gewebe, Körpergewebe, Tressengewebe, Fünfschaft-Atlas-Gewebe oder auch andere Spezialbindungsgewebe. Als Drahtgewebe kommen gemäß einer Ausführungsform der Erfindung Gewebe aus webbaren Metalldrähten, wie Eisen, Federstahl, Messing, Phosphorbronze, Reinnickel, Monel, Aluminium, Silber, Neusilber, Nickel, Chromnickel, Chromstahl, nicht rostenden, säurebeständigen und hochhitzebeständigen Chromnickelstählen sowie Titan in Betracht. Entsprechendes gilt für Gewirke, z.B. Gestricke.

Ebenfalls können Gewebe oder Gestricke aus anorganischen Materialien verwendet werden, wie aus Al₂O₃ und/oder SiO₂.

Auch synthetische Drähte und Gewebe oder Gestricke aus Kunststoffen sind gemäß einer Ausführungsform der Erfindung einsetzbar. Beispiele sind Polyamide, Poly- ester, Polyvinyle, Polyolefine wie Polyethylen, Polypropylen, Polytetrafluorethylen und andere zu Geweben oder Gestricke verarbeitbare Kunststoffe.

Bevorzugte Trägermaterialien sind Metallfolien oder Metallgewebe, wie beispielsweise Edelstähle mit den Werkstoffnummern 1.4767, 1.4401, 2.4610, 1.4765, 1.4847, 1.4301 usw. Die Bezeichnung dieser Werkstoffe mit den genannten Werkstoffnummern folgt den Angaben der Werkstoffnummern in der "Stahleisenliste", herausgegeben vom Verein Deutscher Eisenhüttenleute, 8. Auflage, Seiten 87, 89 und 106, Verlag Stahleisen mbH, Düsseldorf, 1990. Der Werkstoff der Werkstoffnummer 1.4767 ist auch unter dem Namen Kanthal bekannt.

Die Metallfolien und Metallgewebe eignen sich besonders gut, da sie vor der Beschichtung mit katalytischen aktiven Verbindungen bzw. Promotoren durch eine Temperung an der Oberfläche aufgerauht werden können. Dazu werden die metallischen Träger bei Temperaturen von 400 bis 1100°C, vorzugsweise 800 bis 1000°C für 0,5 bis 24 Stunden, vorzugsweise 1 bis 10 Stunden in sauerstoffhaltiger Atmosphäre wie Luft erhitzt. Durch diese Vorbehandlung kann gemäß einer Ausführungsform der Erfindung die Aktivität des Katalysators gesteuert bzw. erhöht werden.

### Beschichtung der Katalysatorträger

Die erfindungsgemäß verwendeten Katalysatorträger können erfindungsgemäß mittels unterschiedlicher Verfahren mit katalytisch aktiven Verbindungen und Promotoren beschichtet werden.

Gemäß einer Ausführungsform der Erfindung erfolgt das Aufbringen der als Katalysator und/oder Promoter aktiven Substanzen durch Tränkung des Trägers in der Substanz, durch elektrochemische Abscheidung oder Abscheidung in Gegenwart eines Reduktionsmittels (stromlose Abscheidung).

Das Katalysatorgewebe oder die Katalysatorfolie kann sodann gemäß einer Ausführungsform der Erfindung für den Einbau in den Reaktor zu Monolithen verformt werden. Gemäß einer weiteren Ausführungsform der Erfindung kann die Verformung auch vor dem Aufbringen der aktiven Substanzen oder Promotoren erfolgen.

Die erfindungsgemäß verwendbaren Katalysatorträger, insbesondere die Gewebe, Gestricke und Folien können gemäß einer Ausführungsform der Erfindung mittels einer Vakuumaufdampftechnik mit "dünnen Schichten" von katalytisch aktiven Verbindungen und Promotoren beschichtet werden. Als "dünne Schichten" werden Belegungen im Dickenbereich zwischen wenigen Å (10⁻¹⁰m) und maximal 0,5 *µ*m bezeichnet. Als Vakuumaufdampftechniken können erfindungsgemäß verschiedene Verfahren angewendet werden. Beispiele sind die thermische Verdampfung, die Flash-Verdampfung, die Kathodenzerstäubung (Sputtern) sowie die Kombination von thermischer Verdampfung und Kathodenzerstäubung. Die thermische Verdampfung kann dabei durch direkte oder indirekte elektrische Beheizung erfolgen.

Eine Verdampfung mittels Elektronenstrahl kann ebenfalls erfindungsgemäß eingesetzt werden. Dazu wird die zu verdampfende Substanz in einem wassergekühlten Tiegel mit einem Elektronenstrahl oberflächlich so stark erhitzt, daß selbst hochschmelzende Metalle und Dielektrika verdampft werden. Durch gezielte Zusätze geeigneter Mengen reaktiver Gase zum Restgas können gemäß einer Ausführungsform der Erfindung beim Schichtaufbau durch Aufdampftechniken chemische Reaktionen bewirkt werden. Durch geeignete Reaktionsführung können somit Oxide, Nitride oder. Carbide auf dem Träger erzeugt werden.

In dem erfindungsgemäßen Verfahren können die Gewebe, Gestricke und Folien in- einer Vakuumbedampfungsanlage diskontinuierlich oder kontinuierlich bedampft werden. Beispielsweise erfolgt die Bedampfung, indem die aufzubringende katalytisch aktive Komponente bzw. Verbindung, beispielsweise ein Edelmetall, im Vakuum bei 1,3·10⁻⁵ bis 1,3·10⁻¹³ bar (10⁻² bis 10⁻¹⁰ Torr) vorzugsweise 1,3·10⁻⁷ bis 1,3·10⁻¹¹ bar (10⁻⁴ bis 10⁻⁸ Torr) mittels eines Elektronenstrahls so stark erhitzt wird, daß das Metall aus dem wassergekühlten Tiegel heraus verdampt und sich auf dem Träger niederschlägt. Das Trägergewebe oder - gestricke wird dabei zweckmäßigerweise so angeordnet, daß ein möglichst großer Teil des Dampfstroms auf dem Träger kondensiert. Durch eine eingebaute Wickelmaschine können die Gewebe oder Gestricke dabei kontinuierlich beschichtet werden. Erfindungsgemäß bevorzugt ist das kontinuierliche Sputtern in einer air to air-Anlage.

Geignete Parameter und Bedingungen der Vakuumaufdampftechniken können beispielsweise dem "Handbook of Thin Film Technology", Verlag Maissel und Glang, McGraw Hill, New York, 1970, "Thin Film Processes" von J.L. Vossen und B. Kern, Academic Press, New York, sowie der

EP-A 0 198 435 entnommen werden. Aus EP-A-0 198 435 geht die Herstellung eines Katalysatornetzpackets durch Bedampfung von Edelstahlgewebe mit Platin bzw. Platin und Rhodium hervor.

Bei der erfindungsgemäßen Herstellung der Katalysatoren durch Vakuumaufdampftechniken sollen möglichst ungeordnete und gestörte polykristalline Teilchen auf dem Träger erzeugt werden, bei denen sich der überwiegende Teil der Atome in der Oberfläche befindet. Somit unterscheidet sie sich von den bekannten Aufdampftechniken in der Optik- und Elektroindustrie, bei denen eine hohe Reinheit der Träger- und Aufdampfmaterialien sichergestellt werden muß und eine vorbestimmte Kondensationstemperatur am Träger sowie eine bestimmte Aufdampfrate eingestellt werden müssen.

Beim erfindungsgemäßen Verfahren können ein oder mehrere katalytisch aktive Verbindungen bzw. Promotoren aufgedampft werden.

Die Beläge mit katalytisch aktiver Substanz liegen gemäß einer Ausführungsform der Erfindung vorzugsweise im Dickenbereich von 0,2 nm bis 100 nm, besonders bevorzugt 0,5 nm bis 20 nm, insbesondere 3 bis 7 nm.

Gemäß einer Ausführungsform der Erfindung werden als katalytisch aktive Verbindungen die Elemente der VIII. Nebengruppe des Periodensystems der Elemente verwendet, vorzugsweise Nickel, Palladium und/oder Platin, insbesondere Palladium. Promotoren können gemäß einer Ausführungsform der Erfindung vorliegen und erfindungsgemäß beispielsweise ausgewählt sein aus den Elementen der III., IV., V., VI. Hauptgruppe sowie der I., II., III., VI., VII. Nebengruppe des Periodensystems der Elemente.

Der Promotor, der gemäß einer Ausführungsform der Erfindung verwendet wird, ist vorzugsweise ausgewählt aus Kupfer, Silber, Gold, Zink, Chrom, Cadmium, Blei, Bismut, Zinn, Antimon, Indium, Gallium, Germanium, Wolfram oder Gemischen davon, besonders bevorzugt Silber, Indium und Germanium, Kupfer, Gold, Zink, Chrom, Cadmium, Blei, Bismut, Zinn, Antimon. Die Schichtdicke des gemäß einer Ausführungsform der Erfindung mindestens einen verwendeten Promotors beträgt 0,1 bis 20 nm, vorzugsweise 0,1 bis 10 nm, insbesondere 0,5 bis 3 nm.

Vor dem Aufbringen der katalytisch aktiven Substanz und/oder des Promotors kann der Träger durch Aufdampfen einer Schicht eines oxidierbaren Metalls und anschließende Oxidation zur Bildung einer Oxidschicht modifiziert werden. Gemäß einer Ausführungsform der Erfindung werden als oxidierbares Metall Magnesium, Aluminium, Silicium, Titan, Zirkonium, Zinn oder Germanium verwendet, wie auch Gemische davon. Die Dicke einer derartigen Oxidschicht liegt erfindungsgemäß im Bereich von 0,5 bis 200 nm, vorzugsweise 0,5 bis 50 nm.

Das beschichte Trägermaterial kann nach dem Beschichten getempert werden, beispielsweise ein mit Palladium beschichtetes Trägermaterial bei Temperaturen von 200 bis 800°C, vorzugsweise 300 bis 700°C für 0,5 bis 2 Stunden.

Nach der Herstellung des Katalysators kann dieser, falls erwünscht oder erforderlich, mit Wasserstoff bei Temperaturen von 20 bis 250°C, vorzugsweise 100 bis 200°C reduziert werden. Diese Reduktion kann auch vorzugsweise im Reaktor selbst durchgeführt werden.

Gemäß einer Ausführungsform der Erfindung können die Katalysatoren systematisch aufgebaut werden, beispielsweise in einer Bedampfungsanlage mit mehreren verschiedenen Verdampfungsquellen. So kann beispielsweise zunächst eine Oxidschicht oder durch reaktives Verdampfen eine Haftschicht auf den Träger aufgebracht werden. Auf diese Grundschicht lassen sich katalytisch aktive Komponenten und Promotoren in mehreren Wechselschichten aufdampfen. Durch Einlaß eines reaktiven Gases in den Rezipienten bei der Aufdampfung können Promotorschichten aus Oxiden und anderen Verbindungen erzeugt werden. Auch Temperschritte können zwischen- oder nachgeschaltet werden.

Das Aufbringen der mindestens einen als Katalysator und Promotor aktiven Substanz kann auch durch Auftränken erfolgen.

Die erfindungsgemäß durch Aufdampfen hergestellten Katalysatoren, insbesondere Katalysatorgewebe, Katalysatorgestricke und Katalysatorfolien weisen eine sehr gute Haftfestigkeit der katalytisch aktiven Verbindungen bzw. Promotoren auf. Deshalb können sie verformt, geschnitten und beispielsweise zu monolithischen Katalystorelementen verarbeitet werden, ohne daß sich die katalytisch aktiven Verbindungen bzw. Promotoren ablösen. Aus den erfindungsgemäßen Katalysatorgeweben, Katalysatorgestricken und Katalysatorfolien lassen sich beliebig geformte Katalysatorpackungen für einen Reaktor, z.B. Durchflußreaktor, eine Reaktionskolonne bzw. Destillationskolonne herstellen. Es lassen sich Katalysatorpackungselemente mit unterschiedlichen Geometrien herstellen, wie sie aus der Destillations- und Extraktionstechnik bekannt sind. Beispiele vorteilhafter erfindungsgemäßer Katalysatorpackungsgeometrien die den Vorteil eines geringen Druckverlusts im Betrieb bieten, sind solche der Bauart Montz A 3 und Sulzer BX, DX und EX. Ein Beispiel einer erfindungsgemäßen Katalysatorgeometrie, aus Katalysatorfolien bzw. Katalysatorstreckmetallfolien sind die des Typs Montz BSH.

Die pro Volumeneinheit verarbeitete Katalysatormenge, insbesondere Katalysatorgewebemenge, Katalysatorgestrickmenge bzw. Katalysatorfolienmenge läßt sich in einem weiten Bereich steuern, wodurch sich eine unterschiedliche Größe der Öffnungen bzw. Kanalbreiten im Katalysatorgewebe, Katalysatorgestrick bzw. in der Katalysatorfolie ergibt. Durch entsprechende Auswahl der Menge an Katalysatorgewebe, Katalysatorgestrick bzw. Katalysatorfolie pro Volumeneinheit kann der maximale Druckverlust im Reaktor, z.B. Durchfluß- oder Destillationsreaktor eingestellt werden und somit der Katalysator an experimentelle Vorgaben angepaßt werden.

Vorzugsweise weist der erfindungsgemäß eingesetzte Katalysator eine Monolith-Form auf, wie sie beispielsweise beschrieben ist in EP-A-0 564 830. Weitere geeignete Katalysatoren sind beschrieben in der EP-A-0 218 124 und der EP-A-0 412 415.

Ein weiterer Vorteil der erfindungsgemäß eingesetzten monolithischen Katalysatoren ist die gute Fixierbarkeit im Reaktorbett, so daß sie beispielsweise bei Hydrierungen in flüssiger Phase in Sumpffahrweise bei hoher Querschnittsbelastung sehr gut eingesetzt werden können. Demgegenüber besteht bei konventionellen Trägerkatalysatoren die Gefahr der Verwirbelung im Katalysatorbett, was zu möglichem Abrieb bzw. Zerfall der Formkörper führen kann. Bei der Gasphasenhydrierung ist die Katalysatorpackung widerstandsfähig bei Stoß oder Vibrationen. Es tritt kein Abrieb auf.

### Hydrierung

Die vorstehend beschriebenen Katalysatoren werden erfindungsgemäß eingesetzt in Verfahren zur selektiven Hydrierung mehrfach ungesättigter C₂₋₆-Kohlenwasserstoffe, insbesondere C₂₋₄-Kohlenwasserstoffe.

Diese mehrfach ungesättigten Kohlenwasserstoffe sind enthalten in C₂-, C₃-, C₄-, C₅- oder C₆-Strömen, aus einem Steamcracker oder katalytischen Cracker. Diese Ströme enthalten in der Regel, wie vorstehend beschrieben, mehr oder weniger große Mengen der entsprechenden mehrfach ungesättigten C₂- bis C₆- Kohlenwasserstoffe.

Durch Einsatz der erfindungsgemäßen Katalysatoren können diese Verbindungen mit hoher Selektivität und hoher Ausbeute in die entsprechenden einfach ungesättigten Kohlenwasserstoffe überführt werden.

Die Selektivhydrierungen werden dabei erfindungsgemäß sowohl in adiabater als auch in isothermer Fahrweise in Gas- und Flüssigphase durchgeführt. Abhängig vom Gehalt der zu hydrierenden Verbindungen im Gasstrom oder Flüssigkeitsstrom ergibt sich die Anzahl der Reaktoren. Beispielsweise genügt für Gehalte unter 1 Gew.-% ein adiabatisch betriebener Reaktor bei Gasphasenhydrierungen, wobei das Wasserstoff/mehrfach ungesättigter Kohlenwasserstoff-Verhältnis etwa 1,8 bis 2 beträgt. Ist der Gehalt an mehrfach ungesättigten Verbindungen höher, so wird die Hydrierung in zwei oder mehr in Serie geschalteten Reaktoren durchgeführt. In diesem Fall wird der Wasserstoff vor jedem Reaktor zugeleitet.

Die Hydrierung eines C₃-Stroms erfolgt dabei in der Gasphase meist in drei hintereinandergeschalteten Reaktoren, wobei im ersten Reaktor ein Umsatz von 60 bis 70% und im zweiten Reaktor ein Umsatz von 30 bis 40% erzielt wird. Im dritten Reaktor wird der Restumsatz erreicht, oder er dient als Sicherheitsreaktor.

Bei der Hydrierung in Flüssigphase genügt ein adiabatisch betriebener Reaktor ohne Rückführung bei Gehalten an mehrfach ungesättigten Kohlenwasserstoffen von bis zu 3,3 Gew.-%. Bei einem Wasserstoff/mehrfach ungesättigter Kohlenwasserstoff-Verhältnis von etwa 1 bis 1,5 erreicht man dabei eine Abreicherung auf 500 bis 1000 ppm im Austrag, was einem Umsatz von 95 bis 99% entspricht. Ist der Gehalt an mehrfach ungesättigten Kohlenwasserstoffen höher, so ist im allgemeinen eine Rückführung erforderlich. Soll eine Verminderung des Gehalts an mehrfach ungesättigten Kohlenwasserstoffen im Austrag auf weniger als 10 ppm erreicht werden, so wird die Hydrierung in der Regel in zwei in Serie geschalteten Reaktoren durchgeführt, wobei wie vorstehend beschrieben der Wasserstoff vor jedem Reaktor zugeleitet wird. Im zweiten Reaktor wird dabei bei einem Wasserstoff/ungesättigter Kohlenwasserstoff-Verhältnis von etwa 4 bis 8 ein Umsatz von mehr als 99,9% insgesamt erreicht.

Bei der Hydrierung von C₂-Strömen mit Acetylengehalten von mehr als 2 Gew.-% wird die Hydrierung üblicherweise in einem isothermen Reaktor und einem oder zwei angeschlossenen adiabatischen Reaktoren durchgeführt.

Bei der Flüssigphasen-Hydrierung eines C₄-Stroms mit hohem Butadiengehalt werden je nach gewünschter Butadienabreicherung eine oder zwei Stufen vorgesehen. Ab einem Abreicherungsfaktor von etwa 200 wird im allgemeinen ein zweistufiges Verfahren bevorzugt. So erfolgt beispielsweise die Selektivhydrierung eines Roh-C₄-Stroms aus einem Steamcracker mit einem Gehalt von etwa 45 Gew.-% Butadien in zwei Stufen auf einen Butadien-Restgehalt von weniger als 10 ppm.

Es ist genauso möglich, niedrige Butadien-Gehalte in einer sogenannten Resthydrierung selektiv zu entfernen. In diesem Fall ist ein einstufiges Verfahren mit Abweichungsfaktoren von über 1000 möglich. So erfolgt beispielsweise die Hydrierung von 0,5 Gew,-% Butadien auf Werte < 10 ppm in einem einstufigen Verfahren, wobei gleichzeitig ein maximaler Erhalt des vorhandenen 1-Butens erreicht wird.

Gemäß einer Ausführungsform der Erfindung wird die Hydrierung in der Gasphase durchgeführt. Insbesondere wird die Hydrierung von C₂- und/oder C₃-Strömen in der Gasphase durchgeführt. Beispiele verwendbarer Reaktoren sind Rohrreaktoren und Schachtreaktoren sowie Rohrbündelreaktoren.

Es können gemäß einer Ausführungsform der Erfindung mehrere Rohrreaktoren hintereinander geschaltet sein. Dabei wird gemäß einer Ausführungsform der Wasserstoff vor jedem Reaktor zugeleitet. Für die weitere Beschreibung erfindungsgemäß geeigneter Reaktoren wird auf die Einleitung verwiesen.

Die selektive Hydrierung in der Gasphase wird gemäß einer Ausführungsform der Erfindung bei Drücken von 5 bis 50 bar, vorzugsweise 10 bis 30 bar, insbesondere 15 bis 25 bar durchgeführt. Die Raumgeschwindigkeiten betragen gemäß einer Ausführungsform der Erfindung 500 bis 8000, vorzugsweise 1000 bis 5000, insbesondere 2000 bis 4000 m³/m³ h. Die Eintrittstemperatur bei der Hydrierung beträgt gemäß einer Ausführungsform der Erfindung -20 bis 150°C, vorzugsweise 20 bis 120°C, insbesondere 20 bis 80°C. Es kann ein adiabatisch oder ein isoterm betriebener Reaktor verwendet werden. Die Hydrierung kann ebenfalls in einer Reihe von hintereinander geschalteten Reaktoren durchgeführt werden, die isothermisch oder adiabatisch betrieben werden. Es können beispielsweise zwei adiabatische Reaktoren auf einen isothermen Reaktor folgen, insbesondere bei Hydrierung eines C₂-Stroms.

Gemäß einer Ausführungsform der Erfindung wird die Hydrierung in der Flüssigphase oder in einer gemischten Flüssig/Gasphase mit mindestens 50 Gew.-% des Kohlenwasserstoffstroms in der Flüssigphase durchgeführt. Dabei kann die Hydrierung gemäß einer Ausführungsform der Erfindung in der Rieselfahrweise oder in der Sumpffahrweise durchgeführt werden. Bei der Sumpffahrweise kann der zugegebene Hydrierwasserstoff in der Flüssigphase gelöst vorliegen. Als Reaktoren können dabei z.B. Rohrreaktoren oder Rohrbündelreaktoren verwendet werden.

Die Hydrierung wird gemäß einer Ausführungsform bei einem Druck von 5 bis 70 bar, vorzugsweise 5 bis 40 bar, insbesondere 10 bis 30 bar durchgeführt. Gemäß einer Ausführungsform der Erfindung beträgt die Raumzeitgeschwindigkeit 1 bis 100, vorzugsweise 2 bis 40, insbesondere 2 bis 20 m³/m³ h. Die Eintrittstemperatur bei der Hydrierung beträgt gemäß einer Ausführungsform der Erfindung -10 bis 150°C, vorzugsweise 0 bis 120°C, insbesondere 0 bis 90°C. Um die Ausbildung einer Flüssigphase zu gewährleisten müssen geeignete Temperatur- und Druckparameter gewählt werden, was abhängig vom jeweilig eingesetzten Stoffgemisch ist.

Gemäß einer Ausführungsform der Erfindung wird die Hydrierung in einem Verfahren zur katalytischen Destillation durchgeführt. Das Verfahren ist dadurch gekennzeichnet, daß die wie vorstehend beschriebene Hydrierung mit einer gleichzeitig Destillation oder Rektifikation an der Katalysatorpackung kombiniert ist.

Es finden dabei gleichzeitig oder unmittelbar aufeinander folgend die Hydrierung und eine Destillation statt. Dabei wird mindestens eine Komponente des Reaktionsgemisches aus dem Hydriergemisch nach der Hydrierung abdestilliert. Unter "katalytische Destillation" versteht man dabei eine chemische Reaktion, hier eine Hydrierung, die in einer geeigneten Vorrichtung kombiniert ist mit einer Destillation oder Rektifikation. Als Reaktor für die katalytische Destillation kann jede geeignete Destillationsapparatur verwendet werden, in der die Katalysatorpackung im Destillationsweg eingebaut werden kann. Dies ist beispielsweise möglich durch Einbau der Katalysatorpackung in eine Destillationskolonne in der Destillationsapparatur.

Das Reaktionsgemisch, d.h. der Kohlenwasserstoffstrom wird dabei an geeigneter Stelle in die Destillationsapparatur eingeführt, gemäß einer Ausführungsform in den Sumpf der Destillationsapparatur. Dies ist insbesondere zweckmäßig bei Hydrierung eines C₃-, C₄-, C₅-, oder C₆-Stroms. Die hydrierten Komponenten und die Alkene werden dabei am Kopf der Destillationsapparatur ausgetragen.

Die Erfindung wird nachstehend durch Beispiele näher erläutert.

Bei den Performancetests zur Hydrierung in der Gasphase wurden die monolithischen Katalysatoren in einer drucklosen Laborapparatur oder in einer Technikumsapparatur unter erhöhten Drücken eingesetzt. Die Eintrittstemperaturen des in die Hydrierzone eintretenden Gasgemisches betragen im allgemeinen 15 bis etwa 120°C, vorzugsweise 25 bis 90°C. Das Volumenverhältnis von Wasserstoff zu den mehrfach ungesättigten Kohlenwasserstoffen beträgt im allgemeinen 0,5:1 bis 2,5:1, bei der C₂-Hydrierung vorzugsweise 1,1:1 bis 2:1, insbesondere 1,2:1 bis 1,8:1 und bei der ersten Stufe der C₃-Hydrierung 0,5:1 bis 0,8:1.

Unter Volumenanteilen an Gas werden im folgenden die Volumenanteile unter Normalbedingungen verstanden.

### Beispiel 1

Drahtgewebe in Leinenbindung aus dem Werkstoff Nr. 1.4301 mit einer Maschenweite von 0,125 mm und einem Drahtdurchmesser von 0,1 mm wurde 3 h bei 800°C an der Luft geglüht. Nach dem Abkühlen wurde das so vorbehandelte Trägergewebe in einer Elektronenstrahlbedampfungsanlage im Vakuum bei 1,3·10⁻⁹ bis 4·10⁻⁹ bar (1 bis 3 x 10⁻⁶ Torr) beidseitig zunächst mit 6 nm Pd und anschließend mit 1 nm Ag bedampft. Die Schichtdicke wurde mittels eines Schwingquarzes gemessen und die Aufdampfrate mit dem Schwingquarz gesteuert. Die aufgedampfte Palladiummenge betrug 138 mg/m² und die Silbermenge 19,5 mg/m². Aus dem so hergestellten Katalysatorgewebe wurden 3 Monolithe von 90 mm Höhe und einem Durchmesser von 18,6 mm geformt. In der Mitte der Monolithe befand sich ein Thermoloch mit einem Durchmesser von 4 mm. Zur Herstellung der Monolithe wurden 92 mm breite und 37,5 cm lange Gewebestreifen geschnitten, von denen der eine mittels einer Zahnradwalze (Modul 0,5 mm) gewellt wurde. Dieses gewellte Gewebe wurde mit dem glatten Gewebe zusammengelegt und um einen 4 mm dicken Metallstab gewickelt. Man erhielt so einen monolithischen Katalysator, der durch Punktschweißen am äußeren Rand gefestigt wurde.

### Beispiel 2

### Gasphasen-Hydrierung eines C₃-Stromes unter Druck

Drei nach Beispiel 1 hergestellte Monolithe mit einer Gesamtoberfläche von 4219 cm² wurden in einem Reaktor zum Test für die Gasphasenhydrierung von Methylacetylen und Propadien in einem C₃-Strom aus einem Steamcracker eingebaut. Axial wurde ein Mehrfach-Thermoelement in das 4 mm starke Thermoloch eingeführt.

Die Verfahrensbedingungen wurden entsprechend den Bedingungen in einer ersten Stufe der meist 3-stufigen selektiven Hydrierung von Methylacetylen und Propadien im C₃-Strom eingestellt.

Der Reaktor hatte einen Durchmesser von 18,6 mm und eine Länge von 2 m. Der Katalysatormonolith mit einer Höhe von 27 cm wies ein Volumen von 70 ml auf.

Nach Spülen mit Stickstoff und Wasserstoff bei 120°C wurden 660 g/h eines Gasgemisches mit der Zusammensetzung 6,8 % Propan, 1,7 % Propadien und 2,2 % Methylacetylen in Propylen mit unterschiedlichen Mengen Wasserstoff vermischt und bei einer Eintrittstemperatur von 50°C und einem Druck von 10 bar über den Katalysator geführt. Die Zusammensetzungen des Reaktionsaustrags sind in der nachstehenden Tabelle zusammengefaßt.

**Tabelle 1**

| **H2 [I/h]** | **H2/MAPD** | **Propan Vol-%** | **Propen Vol-%** | **Propadien Vol-%** | **Propin Vol-%** | **C6+ Vol-%** | **U (MAP D) [%]** | **S(Propen) [%]** |
|---|---|---|---|---|---|---|---|---|
| 8,1 | 0,5 | 6,848 | 91,52 | 0,737 | 0,67 | 0,22 | 64 | 90 |
| 8,9 | 0,55 | 6,895 | 91,61 | 0,653 | 0,594 | 0,244 | 68 | 88 |
| 9,7 | 0,6 | 6,962 | 91,67 | 0,567 | 0,53 | 0,262 | 72 | 86 |
| 11,3 | 0,7 | 7,161 | 91,72 | 0,439 | 0,439 | 0,268 | 78 | 81 |

MAPD bedeutet dabei das Gemisch mehrfach ungesättigter Kohlenwasserstoffe, nämlich Methylacetylen und Propadien. Das Verhältnis von Wasserstoff zu MAPD ist das Volumenverhältnis. U bedeutet den Umsatz, S die Selektivität in bezug auf Propen.

Der Katalysator weist unter den Bedingungen der ersten Hydrierstufe sehr hohe Selektivitäten auf.

### Beispiel 3

Drahtgewebe in Leinenbindung aus dem Werkstoff Nr. 1.4767 mit einer Maschenweite von 0,18 mm und einem Drahtdurchmesser 0,112 mm wurde 5 Stunden bei 900°C an der Luft geglüht. Nach dem Abkühlen wurde das so vorbehandelte Trägergewebe in einer Elektronenstrahlbedampfungsanlage in Vakuum bei 1,3·10⁻⁹ bar (1 x 10⁻⁶ Torr) beizeitig zunächst mit 92 mg Pd/m² und anschließend unter gleichen Bedingungen mit 26,4 mg Zn/m² bedampft. Wie in Beispiel 1 beschrieben wurde aus dem so erhaltenen Katalysatorgewebe ein gewickelter Monolith von 126 cm³ hergestellt.

### Beispiel 4 (Referenz)

### Drucklose C₂-Gasphasenhysrierung

Der nach Beispiel 3 erhaltene Katalysatormonolith wurde in einem Rohrreaktor eingebaut, wie er in Beispiel 2 beschrieben ist. Der Test des Katalysators erfolgte drucklos mit einem Gasgemisch aus 1 Vol.-% Acetylen, 2 Vol.-% Wasserstoff und 97 Vol.-% Ethylen bei einer Belastung von 3000 m³/m³(Kat) h. Bei einer Temperatur von 82°C wurde ein Acetylenumsatz von 70 % bei einer Selektivität zu Ethylen von 97 % erzielt. Unter sonst gleichen Reaktionsbedingungen lieferte ein handelsüblicher Trägerkatalysator mit 0,02 Gew.-% Pd und 0,01 Gew.-% Zn bei einem Umsatz von 70 % nur eine Selektivität zu Ethylen von 62 %.

### Beispiel 5 (Referenz)

Als Trägermaterial wurde das in Beispiel 3 beschriebene Material verwendet, das durch Erhitzen auf 900°C an der Luft vorbehandelt und anschließend analog Beispiel 3 mit 138 mg/m² Palladium bedampft wurde. Durch Zusammenrollen eines gewellten und eines glatten Katalysatorgewebestreifens von 10 cm Breite wurde ein Monolith mit 5 mm Thermoloch hergestellt. Der erhaltene Monolith hatte ein Volumen von 71,6 cm³ und bestand aus 15,25 dm² Katalysatorgewebe.

### Beispiel 6 (Referenz)

### Gasphasenhydrierung eines C₂-Stromes unter Druck von 20 bar

Der gemäß Beispiel 5 hergestellte Katalysatormonolith wurde in einen Rohrreaktor eingebaut, wie er im Beispiel 2 beschrieben ist. Nach Spülung mit Stickstoff wurde der Katalysator für 3 Stunden bei 150°C mit 10 l/h Wasserstoff reduziert. Bei einer Eintrittstemperatur von 82°C wurde der Katalysator dann mit 160 l/h eines Gasgemisches aus 98,824 Vol.-% Ethylen und 1,145 Vol.-% Acetylen, das mit 1,46 Vol.-% Wasserstoff vermischt wurde, belastet. Der Austrag enthielt 99,394 Vol.-% Ethylen, 0,486 Vol.-% Ethan und 0,01 Vol.-% Acetylen (Umsatz 99,1%, Selektivität 58 %).

Bei Erhöhung des Wasserstoffgehalts auf 1,67 Vol.-% stieg der Ethan-Gehalt im Austrag auf 0,678 Vol.-%. Acetylen war dann nicht mehr nachweisbar (Umsatz 100 %, Selektivität 43 %).

Durch Zugabe von 1,5 ppm Kohlenmonoxid konnte die Selektivität weiter gesteigert werden. Bei einer Eintrittstemperatur von 84°C wurde ein Austrag erhalten, der acetylenfrei war. Der Ethylengehalt lag bei 99,419 Vol.-%, der Ethangehalt bei 0,442 Vol.-%.

### Beispiel 7

Auf ein Trägergewebe aus dem Werkstoff Nr. 1.4767, welches wie in Beispiel 1 beschrieben durch Erhitzen an der Luft bei 900°C vorbehandelt wurde, wurden gemäß dem in Beispiel 1 beschriebenen Verfahren 138 mg Pd/m² und danach 19,5 mg Ag/m² aufgedampft. Anschließend wurde aus dem Katalysatorgewebe ein Monolith mit einem Volumen von 126 cm³ gefertigt.

### Beispiel 8 (Referenz)

### Drucklose Gasphasenhydrierung eines C₂-Stromes

Der nach Beispiel 7 hergestellte Katalysator wurde wie in Beispiel 4 beschrieben für die selektive Acetylenhydrierung eingesetzt. Bei einem Umsatz von 70 % wurde eine Selektivität zu Ethylen von 91 % erzielt.

### Beispiel 9

### Flüssigphasenhydrierung von C₃-Strömen

Der gemäß Beispiel 1 hergestellte Katalysator wurde für die Flüssigphasenhydrierung von Methylacetylen und Propadien in einem C₃-Strom aus einem Steamcracker verwendet. Die Verfahrensbedingungen wurden entsprechend den Bedingungen einer ersten Stufe der meist 2-stufigen Hydrierung im C₃-Strom gewählt.

In einem adiabatisch betriebenen Rohrreaktor von einem Durchmesser von 20 mm wurden 3 der nach Beispiel 1 hergestellten Monolithe mit einer Gesamtoberfläche von 4219 cm² eingebaut. Axial wurde ein Mehrfachthermoelement in das 4 mm starke Thermoloch eingeführt. Um eine gute Benetzung des Katalysators zu gewährleisten, wie sie in den technischen Reaktoren durch die hohe Querschnittsbelastung gegeben ist, wurde Sumpffahrweise gefahren. Nach Spülen mit Stickstoff und mit Wasserstoff bei 120°C wurden 520 g/h eines C₃-Stromes aus einem Steamcracker mit der Zusammensetzung 6,8 Vol.-% Propan, 1,7 Vol.-% Propadien und 2,2 Vol.-% Methylacetylen in Propylen und 13 Nl/h Wasserstoff bei einer Eintrittstemperatur von 10°C und einem Druck von 23 bar über den Katalysator geführt. Der Reaktionsaustrag bestand aus 7,3 Vol.-% Propan, 0,3 Vol.-% Unbekannten (Oligomere) in Propylen. Dies entspricht einer Selektivität zu Propylen von 80 % bei einem Umsatz von mehr als 99,9 %. Die Ergebnisse sind in Tabelle 2 zusammengefaßt.

### Vergleichsbeispiel 1

Der nach EP-A-0 653 243 hergestellte Katalysator wurde als Vergleichskatalysator in der Flüssigphasenhydrierung von Methylacetylen und Propadien im C₃-Strom aus einem Steamcracker eingesetzt. Die Verfahrensbedingungen wurden wie in Beispiel 9 gewählt. In den adiabatisch betriebenen Rohrreaktor wurden 70 ml des Katalysators eingebaut. Nach Spülen mit Stickstoff und Wasserstoff bei 120°C wurden 520 g/h eines C₃-Stromes aus einem Steamcracker mit der Zusammensetzung 5,1 Vol.-% Propan, 1,8 Vol.-% Propadien, 2,3 Vol.-% Methylacetylen in Propylen und 13 Nl/h Wasserstoff bei einer Eintrittstemperatur von 10°C und einem Druck von 22 bar über den Katalysator geführt. Der Reaktionsaustrag bestand aus 5,5 Vol.-% Propan, 0,5 Vol.-% Unbekannte in Propylen. Bei den Unbekannten handelt es sich um gebildete Oligomere. Dies entspricht einer Selektivität zu Propylen von 78 % bei einem Umsatz größer als 99,9 %. Die Ergebnisse sind in Tabelle 2 zusammengefaßt.

### Vergleichsbeispiel 2

Der in Chem. Eng. Prog., 70 (1974) 74 bis 80 beschriebene Katalysator LD 265 wurde als Vergleichskatalysator für die Flüssigphasenhydrierung von Methylacetylen und Propadien in einem C₃-Strom aus einem Steamcracker unterzogen. Die Verfahrensbedingungen wurden wie in Vergleichsbeispiel 1 gewählt, jedoch enthielt der Strom 8 % Propan, 1,7 Vol.-% Propadien und 2,1 Vol.-% Methylacetylen. Der Reaktionsaustrag bestand aus 8,5 Vol.-% Propan, 0,7 Vol.-% Unbekannten in Propylen. Dies entspricht einer Selektivität zu Propylen von 69 % bei einem Umsatz von mehr als 99,9 %. Die Ergebnisse sind in Tabelle 2 zusammengefaßt.

**Tabelle 2**

| **Katalysator** | **Vergl. Bsp. 1 (0,3% Pd, 0,4 kg/l)** | **Vergl. Bsp. 2 (0,3% Pd, 0,7 kg/l)** | **Bsp. 9 Pd/Ag-Kat.** |
|---|---|---|---|
| whsv [kg/l] | ca. 6,5 | ca. 6,5 | ca. 6,5 |
| Druck [bar] | 22 | 22 | 22 |
| H₂/MAPD ber. [mol/mol] | 1,08 | 1,1 | 1,1 |
| Tₑᵢₙ [°C] | 10 | 10 | 10 |
| MAPD [ppm] | <10 | <10 | <10 |
| S-Propen [%] | 78 | 69 | 80 |
| U [%] | >99,9 | >99,9 | >99,9 |
| Δ-Propan [%] | 0,5 | 0,5 | 0,5 |
| Δ-Unbek. [%] | 0,5 | 0,7 | 0,3 |

whsv bedeutet in der Tabelle die stündliche Gewichtsraumgeschwindigkeit in kg/l angegeben. MAPD bedeutet die Menge an mehrfach ungesättigten Kohlenwasserstoffen, nämlich Methylacetylen und Propadien. Die angegebenen Verhältnisse H₂/MAPD wurden aus den für die Reaktion verbrauchten H₂-Mengen berechnet.

Aus der Tabelle läßt sich eine Steigerung der Selektivität von Vergleichsbeispiel 2 über Vergleichsbeispiel 1 zu Beispiel 9 feststellen. Obwohl der Dünnschichtkatalysator gemäß Beispiel 9 nur 28 mg Pd und 4 mg Ag in der eingesetzten Menge Katalysator enthält und beispielsweise der Katalysator aus Vergleichsbeispiel 2 240 mg Pd enthält, zeigt er eine vergleichbare Aktivität und eine höhere Selektivität. Die Bildung von unter Unbekannten zusammengefaßten Oligomeren ist beim erfindungsgemäßen Katalysator gemäß Beispiel 9 am geringsten.

### Beispiel 10

Zur Herstellung des Katalysators wurde Drahtgewebe in Leinenbindung aus dem Werkstoff Nr. 1.4767 mit einer Maschenweite 0,18 mm und einem Drahtdurchmesser von 0,112 mm für 5 Stunden bei 900°C an der Luft erhitzt. Nach dem Abkühlen wurde das Trägermaterial im Vakuum bei 1,3·10⁻⁹ bar (1 x 10⁻⁶ Torr) beidseitig mit 138 mg Pd/m² Gewebe bedampft. Anschließend wurden aus diesem Katalysatorgewebe Monolithe hergestellt. Dazu wurde ein 20 cm breiter Gewebestreifen mittels einer Zahnradwalze (Modul 0,5 mm) gewellt und mit einem glatten Gewebe um einen Metallstab von 4,5 mm Durchmesser zu einer Rolle aufgewickelt. Die Rolle wurde durch Punktschweißen am äußeren Rand befestigt und der Metallstab für das Thermoloch entfernt. Der so erhaltene monolitische Katalysator besaß einen Durchmesser von 16 mm und eine Höhe von 20 cm. Die Katalysatorgewebemenge für einen Monolithen betrug 940 cm². In den Hydrierreaktor wurden 5 Monolithe eingebaut.

### Beispiel 11

### Flüssigphasenhydrierung von Roh-C₄-Schnitt aus einen Steamcracker

Die Selektivhydrierung von Roh-C₄-Schnitt erfolgte an dem Katalysator aus Beispiel 10 in einem Festbettreaktor einer Pilot Plant Unit, welche mit einem Abscheider und Flüssigkeitskreislauf ausgestattet war. Der mittels einer Elektrobeheizung beheizbare Festbettreaktor hatte einen Durchmesser von 16 mm und eine Länge von 2 m. Das Einsatzprodukt wurde über eine Förderpumpe dem Kreislaufstrom zudosiert und an einer Mischstelle mit dem erforderlichen Wasserstoff vermischt. Die Selektivhydrierung erfolgte im Festbett, wobei der im Beispiel 10 beschriebene monolithische Katalysator eingesetzt wurde. Das Reaktionsgemisch gelangte anschließend in einen Abscheider, in dem eine Trennung der Gas- und Flüssigphase erfolgte. Der Großteil der Flüssigphase wurde im Kreislauf geführt. Ein kleinerer, der Menge des Einsatzprodukts entsprechender Teil wurde kontinuierlich aus dem System ausgeschleust und der Austrag gaschromatographisch analysiert.

Vor Beginn des Versuchs wurde der eingebaute monolithische Katalysator für 12 Stunden mit Wasserstoff bei 120°C und 5 bar Druck reduziert. Anschließend wurde die Anlage mit hydriertem C₄-Schnitt und Wasserstoff angefahren. Die Ergebnisse des Versuchs zur Selektivhydrierung sind in der nachstehenden Tabelle 3 zusammengefaßt.

**Tabelle 3**

| | **Einsatzprodukt** | **Pd-Katalysator aus Beispiel 10** | |
|---|---|---|---|
| Raumzeitgeschwindigkeit [m³/m³h] | | 9,0 | 9,0 |
| Recycle/Feed | | 8,2 | 8,2 |
| Tᵢₙ [°C] | | 60 | 60 |
| p [bar] | | 17,7 | 18,3 |
| Verhältnis H₂/(Butadien+Butenin+Butin) | | 0,98 | 1,02 |
| Butadien+Butenin+Butin [Gew.-%] | 34,9 | 1,8 | 0,5 |
| 1-Buten [Gew.-%] | 14,2 | 40,3 | 39,5 |
| 2-trans-Buten [Gew.-%] | 4,5 | 17,6 | 18,6 |
| 2-cis-Buten [Gew.-%] | 3,3 | 5,7 | 6,2 |
| i-Buten [Gew.-%] | 23,6 | 23,6 | 23,6 |
| i-Butan [Gew.-%] | 3,0 | 3,0 | 3,0 |
| n-Butan [Gew.-%] | 7,2 | 7,7 | 8,3 |
| C₅-Kohlenwasserstoffe [Gew.-%] | 0,3 | 0,3 | 0,3 |
| Umsatz [%] | | 95,9 | 98,9 |
| Ges.-Buten-Selektivität [%] | | 98,8 | 97,5 |

Der Katalysator zeigte eine sehr hohe Aktivität. Es konnten auch bei hohen Raumzeitgeschwindigkeiten hohe Umsätze erzielt werden. Selbst bei einer Hydrierung auf einen Butadien-Restgehalt von 1,8 Gew.-% betrug die Hydrierung zu n-Butan nur 0,5 Gew.-%. Es fand keine Hydrierung des i-Buten statt.

### Beispiel 12

Zur Herstellung des Katalysators wurde Drahtgewebe in Leinenbindung aus den Werkstoff Nr. 1.4767 mit einer Maschenweite von 0,18 mm und einem Drahtdurchmesser von 0,112 mm 5 Stunden bei 1000°C an der Luft erhitzt. Nach dem Abkühlen wurde das Trägermaterial im Vakuum bei 1,3·10⁻⁹ bar (1 x 10⁻⁶ Torr) beidseitig mit 92 mg Pd/m² bedampft. Zur Selektivitätssteigerung wurde das Pd Katalysatorgewebe anschließend mit 0,5 nm Germanium durch Aufdampfung dotiert. Die Schichtdicke der Germaniumdotierung wurde dabei mit einem Schwingquarz während des Aufdampfvorgangs gemessen. Aus dem so gewonnenem Katalysatorgewebe wurden entsprechend Beispiel 10 5 Monolithe gefertigt und in den Hydrierreaktor eingebaut.

### Beispiel 13

### Flüssigphasenhydrierung von Roh-C₄-Schnitt aus einem Steamcracker

Der in Beispiel 12 beschriebene Katalysator wurde ebenfalls in der in Beispiel 11 beschriebenen Anlage eingesetzt. Vor Beginn des Versuchs erfolgte analog Beispiel 11 eine 12-stündige Reduktion mit Wasserstoff bei 120°C und 5 bar Druck. Die Anlage wurde anschließend mit hydriertem C₄-Schnitt und Wasserstoff angefahren. Die Ergebnisse des Versuchs zur Selektivhydrierung sind in der nachstehenden Tabelle 4 zusammengefaßt.

**Tabelle 4**

| | **Einsatzprodukt** | **Pd/Ge-Katalysator aus Beispiel 12** |
|---|---|---|
| Raumzeitgeschwindigkeit [m³/m³h] | | 9,0 |
| Recycle/Feed | | 8,2 |
| Tᵢₙ [°C] | | 60 |
| p [bar] | | 17,2 |
| Verhältnis H₂/(Butadien+Butenin+Butin) | | 0,97 |
| Butadien+Butenin+Butin [Gew.-%] | 46,4 | 2,4 |
| 1-Buten [Gew.-%] | 15,2 | 42,5 |
| 2-trans-Buten [Gew.-%] | 5,1 | 18,9 |
| 2-cic-Buten [Gew.-%] | 3,8 | 6,3 |
| i-Buten [Gew.-%] | 23,9 | 23,9 |
| i-Butan [Gew.-%] | 1,0 | 1,0 |
| n-Butan [Gew.-%] | 4,4 | 4,8 |
| C₅-Kohlenwasserstoffe [Gew.-%] | 0,2 | 0,2 |
| Umsatz [%] | | 94,8 |
| Ges.-Buten-Selektivität [%] | | 99,1 |

Der Katalysator wies eine sehr hohe Aktivität auf. Bei seinem Einsatz war die Erzielung hoher Raumzeitgeschwindigkeiten bei gleichzeitig hohem Umsatz möglich. Gegenüber dem Katalysator aus Beispiel 11 ist die Gesamtbuten-Selektivität etwas verbessert und liegt bei über 99 %. Es fand keine Hydrierung des i-Butens statt.

### Beispiel 14

Analog Beispiel 12 wurde Metallgewebe des Werkstoffs Nr. 1.4767 für 5 Stunden bei 1000°C an der Luft geglüht. Nach dem Abkühlen wurde in der beschriebenen Vakuumbeschichtungsanlage das Trägergewebe mit 50 nm Mg beschichtet. Die Schichtdicke wurde dabei mit einem Schwingquarz während des Aufdampfvorgangs gemessen. Anschließend wurde das Gewebe in 60 min auf 300°C erhitzt und an Luft 30 min bei dieser Temperatur belassen. Nach erneutem Einbau in die Beschichtungsanlage wurde bei 1,3·10⁻⁹ bar (1 x 10⁻⁶ Torr) mit 6 nm Pd beschichtet. Aus dem so gewonnenen Katalysatorgewebe wurden analog Beispiel 10 5 Monolithe gefertigt und in den Hydrierreaktor eingebaut.

### Beispiel 15

### Flüssigphasenhydrierung von Roh-C₄-Schnitt aus einem Steamcracker

Der gemäß Beispiel 14 hergestellte Katalysator wurde ebenfalls in der Beispiel 11 beschriebenen Anlage getestet. Vor Beginn des Versuchs erfolgte analog Beispiel 11 eine 12-stündige Reduktion mit Wasserstoff bei 100°C und 5 bar Druck. Die Anlage wurde anschließend mit hydriertem C₄-Schnitt und Wasserstoff angefahren. Die Ergebnisse des Versuchs zur Selektivhydrierung sind in der nachstehenden Tabelle 5 zusammengefaßt.

**Tabelle 5**

| | **Einsatzprodukt** | **Pd/MgO-Katalysator aus Beispiel 14** |
|---|---|---|
| Raumzeitgeschwindigkeit [m³/m³h] | | 9,0 |
| Recycle/Feed | | 8,2 |
| Tᵢₙ [°C] | | 60 |
| p [bar] | | 16,3 |
| Verhältnis H₂/(Butadien+Butenin+Butin) | | |
| Butadien+Butenin+Butin [Gew.-%] | 44,1 | 2,9 |
| 1-Buten [Gew.-%] | 14,2 | 39,7 |
| 2-trans-Buten [Gew.-%] | 4,6 | 17,4 |
| 2-cic-Buten [Gew.-%] | 3,3 | 5,8 |
| i-Buten [Gew.-%] | 23,6 | 23,9 |
| i-Butan [Gew.-%] | 2,9 | 2,9 |
| n-Butan [Gew.-%] | 7,1 | 7,5 |
| C₅-Kohlenwasserstoffe [Gew.-%] | 0,2 | 0,2 |
| Umsatz [%] | | 93,4 |
| Ges.-Buten-Selektivität [%] | | 99,0 |
| | | 0,97 |

Der Katalysator zeigte ebenfalls eine hohe Aktivität und ermöglichte eine hohe Raumzeitgeschwindigkeit bei gleichzeitig hohem Umsatz. Die Leistungsdaten sind ähnlich denen des Katalysators aus Beispiel 13. Es fand keine Hydrierung des i-Butens statt.

Wie aus den Beispielen hervorgeht, sind die erfindungsgemäßen Katalysatoren sehr gut geeignet zur selektiven Hydrierung von mehrfach ungesättigten Kohlenwasserstoffen.

### Beispiel 16

Zur Herstellung des erfindungsgemäßen Katalysators wurde Drahtgewebe in Leinenbindung aus dem Werkstoff Nr. 1.4301 mit einer Maschenweite von 0,180 mm und einem Drahtdurchmesser von 0,105 mm 3 h bei 800°C an der Luft geglüht. Nach dem Abkühlen wurde das so vorbehandelte Trägergewebe mit 5nm Pd und 1 nm Ag im Rollcoater durch Sputtern beschichtet.
Anschließend wurden aus dem Katalysatorgewebe Monolithe hergestellt. Dazu wurde ein 20 cm breiter Gewebestreifen mittels einer Zahnradwalze (Modul 0,5 mm) gewellt, und es wurden analog Beipiel 10 fünf Monolithe mit einem Durchmesser von 16 mm, einer Höhen von 20 cm und einem inneren Thermoloch mit einem Durchmesser von 4,5 mm hergestellt. Die Katalysatorgewebemenge für einen Monolithen betrug 1180 cm². Die fünf Monolithe wurden schließlich in den Hydrierreaktor, welcher im Beipiel 11 beschrieben ist, eingebaut.

Vor Beginn des Versuchs wurde der erfindungsgemäße Pd,Ag-Katalysator für 12 Stunden mit Wasserstoff bei 120°C und 5 bar Druck reduziert. Anschließend wurde die Anlage mit Butadien-haltigem Raffinat 1 und Wasserstoff angefahren. Die Ergebnisse dieses Versuchs sind in Tabelle 6 zusammengefaßt.

### Beipiel 17

Zur Herstellung des erfindungsgemäßen Katalysators wurde Drahtgewebe in Leinenbindung aus dem Werkstoff Nr. 1.4767 mit einer Maschenweite von 0,18 mm und einem Drahtdurchmesser von 0,112 mm 5 h bei 900°C an der Luft geglüht. Nach dem Abkühlen wurde das so vorbehandelte Trägergewebe im Vakuum bei 1,3·10⁻⁹ bar (1 x 10⁻⁶ torr) beidseitig zunächst mit 4 nm Pd und anschließend mit 2 nm Ag bedampft. Die Schichtdicke wurde mittels eines Schwingquarzes gemessen un die Aufdampfrate mit dem SChwingquarz gesteuert. Anschließend wurden aus diesem Katalysatorgewebe Monolithe hergestellt. Dazu wurde ein 20 cm breiter Gewebestreifen mittels einer Zahnradwalze (Modul 0,5 mm) gewellt, und es wurden analog Beispiel 10 fünf Monolithe mit einem Durchmesser von 16 mm, einer Höhe von 20 cm und einem inneren Thermoloch mit einem Durchmesser von 4,5 mm hergestellt. Die Katalysatorgewebemenge für einen Monolithen betrug 940 cm². Die fünf Monolithe wurden schließlich in den Hydrierreaktor, welcher im Beispiel 11 beschrieben ist, eingebaut.

Vor Beginn des Versuchs wurde der erfindungsgemäße Pd, Ag-Katalysator für 12 Stunden mit Wasserstoff bei 120°C und 5 bar Druck reduziert. Anschließend wurde die Anlage mit Butadien-haltigem Raffinat 1 und Wasserstoff angefahren. Die Ergebnisse dieses Versuchs sind in Tabelle 6 zusammengefaßt.

Die Tabelle 6 zeigt einen Performance-Vergleich zwischen einem konventionellen Katalysator und den beiden erfindungsgemäßen Katalysatoren aus Beispielen 16 und 17. Wie zu erkennen ist, zeigt der erfindungsgemäße Katalysator aus Beipiel 16 gegenüber dem beschriebenen Vergleichskatalysator bei gleichem Butadien-Endgehalt im hydrierten Produkt von 20 ppm einen um etwa 3% höheren 1-Buten-Erhalt. Wesentlich ausgeprägter treten die Vorteile des erfindungsgemäßen, monolithischen Katalysators aus Beipiel 17 hervor, in dem ein Butadien-Restgehalt im hydrierten Produkt von 10 ppm erreicht wurde. Der erreichte 1-Buten-Erhalt betrug hierbei über 97%.

Aus den Performance-Daten lassen sich gegenüber dem beschriebenen Vergleichskatalysator vier signifikante Vorteile des erfindungsgemäßen Katalysators herauslesen:
(i) kleineres H₂/Butadien-Verhältnis
   (1,6 anstatt 1,9 beim Vergleichskatalysator)
(ii) geringere Überhydrierung zu n-Butan
   (0,4 Gew.-% n-Butanbildung anstatt 0,8 Gew.-% beim Vergleichskatalysator)
(iii) wesentlich höherer 1-Buten-Erhalt
   97,4 % anstatt 89,3% beim Vergleichskatalysator)
(iv) wesentlich geringerer Aktivkomponentengehalt bei hoher Aktivität
   (12,3 mg Aktivkomponente in eingesetzter Menge Katalysator anstatt 480 mg beim Vergleichskatalysator)

Bei allen Beispielen konnte keine Hydrierung des i-Butens festgestellt werden.

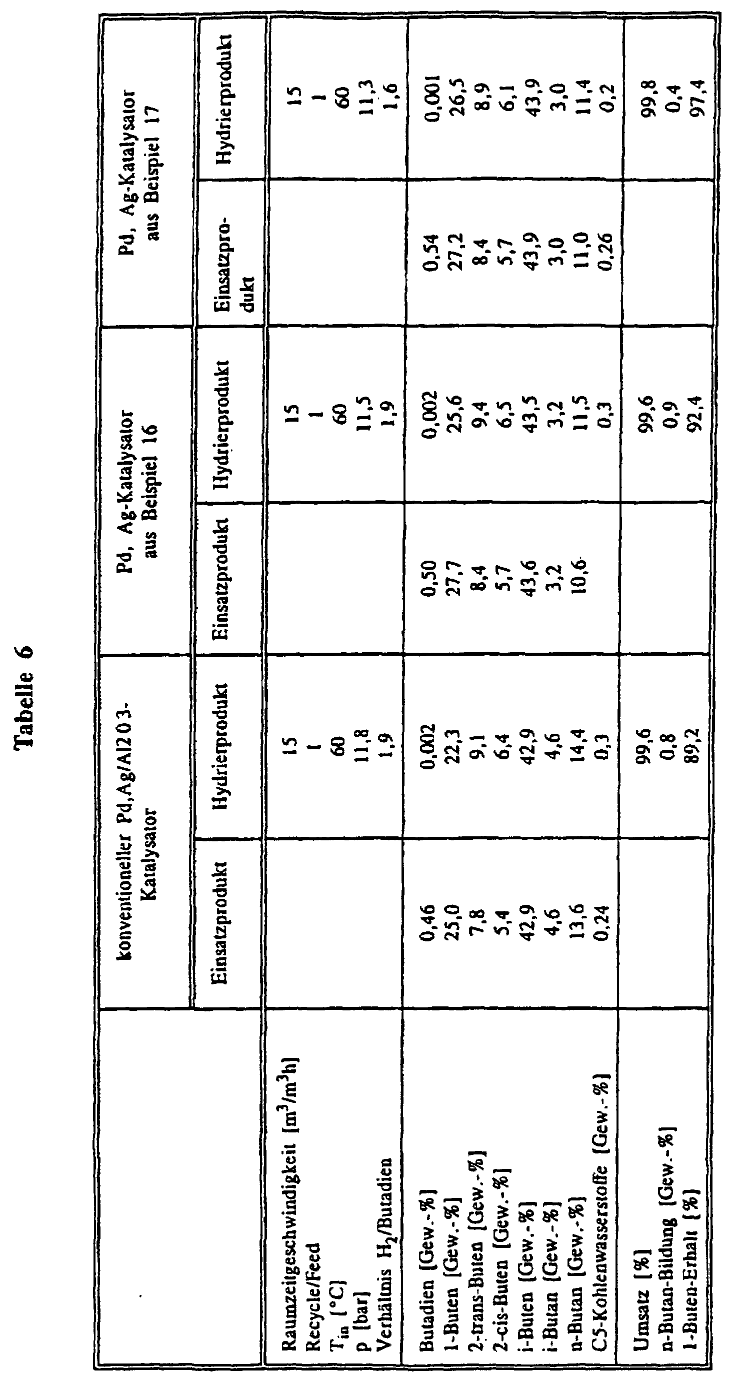

### Beispiel 18

Der konventionelle Vergleichskatalysator und der in Beipiel 17 beschriebene erfindungsgemäße Katalysator wurden ebenfalls unter schärferen Hydrierbedingungen in der in Beispiel 11 beschriebenen Pilot Plant Unit getestet, unter denen ein Butadien-Restgehalt im hydrierten Produkt von < 10 ppm erreicht werden konnte. Die erhaltenen Ergebnisse sind in Tabelle 7 zusammengefaßt.

**Tabelle 7**

| | konventioneller Pd,Ag/Al2O3-Katalysator | | Pd,Ag-Katalysator aus Beispiel 17 | |
|---|---|---|---|---|
| | Einsatzprodukt | Hydrierprodukt | Einsatzprodukt | Hydrierprodukt |
| Raumzeitgeschwindigkeit [m³/m³h] | | 15 | | 15 |
| Recycle/Feed | | 1 | | 1 |
| Tᵢₙ [°C] | | 60 | | 60 |
| p [bar] | | 11,9 | | 11,3 |
| Verhältnis H₂/Butadien | | 2,7 | | 2,1 |
| Butadien [Gew.-%] | 0,43 | < 0,001 | 0,54 | < 0,001 |
| 1-Buten [Gew.-%] | 25,1 | 20,8 | 27,2 | 25,9 |
| 2-trans-Buten [Gew.-%] | 7,9 | 10,2 | 8,4 | 9,0 |
| 2-cis-Buten [Gew.-%] | 5,4 | 7,1 | 5,7 | 6,3 |
| i-Buten [Gew.-%] | 42,2 | 42,2 | 43,9 | 43,9 |
| i-Butan [Gew.-%] | 4,7 | 4,7 | 3,0 | 3,0 |
| n-Butan [Gew.-%] | 14,0 | 14,8 | 11,0 | 11,7 |
| C5-Kohlenwasserstoffe [Gew.-%] | 0,27 | 0,2 | 0,26 | 0,2 |
| Umsatz [%] | | > 99,8 | | > 99,8 |
| n-Butan-Bildung [Gew.-%] | | 0,8 | | 0,7 |
| 1-Buten-Erhalt [%] | | 82,9 | | 95,2 |

Auch bei einer Hydrierung auf Butadienwerte < 10 ppm zeigt der erfindungsgemäße Katalysator die bereits erwähnten Vorteile eines kleinen H2/Butadien-Verhältnisses, einer geringeren Überhydrierung zu n-Butan und eines wesentlich höheren 1-Buten-Erhalts. Analog den vorherigen Beispielen konnte auch in diesem Fall keine Hydrierung des i-Butens festgestellt werden.

## Patentansprüche

1. Verfahren zur selektiven Hydrierung von mehrfach ungesättigten C₂₋₆-Kohlenwasserstoffen, in diese enthaltenden C₂-, C₃-, C₄-, C₅- oder C₆-Strömen aus einem Steamcracker oder einem katalytischen Cracker zu den entsprechenden einfach ungesättigten Kohlenwasserstoffen durch Kontaktieren mit einer Katalysatorpackung in Gegenwart von freiem Wasserstoff, **dadurch gekennzeichnet, daß** die Katalysatorpackung herstellbar ist durch Aufbringen mindestens einer als Katalysator und gegebenenfalls mindestens einer als Promotor aktiven Substanz auf Gewebe, Gestricke oder Folien als Trägermaterial.

2. Verfahren nach Anspruch 1, **dadurch gekennzeichnet, daß** das Aufbringen der mindestens einer als Katalysator und gegebenenfalls mindestens einer als Promotor aktiven Substanz durch Aufdampfen und/oder Sputtern oder durch Auftränken erfolgt.

3. Verfahren nach Anspruch 1 oder 2, **dadurch gekennzeichnet, daß** die Katalysatorpackung aus mindestens einem Monolithen besteht, der aus dem Gewebe, Gestricke oder der Folie, die in Form eines Bandes vorliegen, gebildet wird.

4. Verfahren nach einem der Ansprüche 1 bis 3, **dadurch gekennzeichnet, daß** das Gewebe, das Gestricke bzw. die Folie aus Metall, oder anorganischem Material besteht.

5. Verfahren nach Anspruch 4, **dadurch gekennzeichnet, daß** das Gewebe, das Gestricke oder die Folie aus Metall vor dem Aufdampfen und/oder Sputtern für 0,5 bis 24 Stunden in sauerstoffhaltiger Atmosphäre auf eine Temperatur im Bereich von 400 bis 1100°C erhitzt wird.

6. Verfahren nach einem der Ansprüche 1 bis 5, **dadurch gekennzeichnet, daß** die als Katalysator aktiven Substanzen ausgewählt sind aus den Elemten der I. und/oder VII. und/oder VIII. Nebengruppe und/oder die Promotoren ausgewählt sind aus den Elementen der III., IV., V., VI. Hauptgruppe, II., III., VI., VII. Nebengruppe des Periodensystems der Elemente.

7. Verfahren nach einem der Ansprüche 1 bis 6, **dadurch gekennzeichnet, daß** die Hydrierung von C₂- und/oder C₃-Strömen in der Gasphase durchgeführt wird.

8. Verfahren nach einem der Ansprüche 1 bis 6, **dadurch gekennzeichnet, daß** die Hydrierung von C₃-, C₄-, C₅- und/oder C₆-Strömen in der Flüssigphase oder in einer gemischten Flüssig/Gasphase mit mindestens 50 Gew.-% des Fluids in der Flüssigphase durchgeführt wird.

9. Verfahren zur katalytischen Destillation, **dadurch gekennzeichnet, daß** eine Hydrierung, wie sie in einem der Verfahrensansprüche 1 bis 6 definiert ist, mit einer gleichzeitigen Destillation oder Rektifikation an der Katalysatorpackung kombiniert ist, wobei die Fluide C₃-, C₄-, C₅- und/oder C₆-Ströme sein können.

## Claims

1. A process for the selective hydrogenation of multiply unsaturated C₂-C₆ hydrocarbons C₂, C₃, C₄, C₅ or C₆ streams comprising these from a steam cracker or a catalytic cracker to give the corresponding singly unsaturated hydrocarbons by contact with a catalyst packing in the presence of free hydrogen, wherein the catalyst packing can be produced by applying at least one substance which is active as catalyst and possibly at least one substance active as promoter to woven meshes, knitted fabrics or foils as support material.

2. A process as claimed in claim 1, wherein the at least one substance active as catalyst and possibly at least one substance active as promoter are applied by vapor deposition and/or sputtering or by impregnation.

3. A process as claimed in claim 1 or 2, wherein the catalyst packing comprises at least one monolith which is fabricated from the woven mesh, knitted fabric or foil which is in the form of a strip.

4. A process as claimed in any of claims 1 to 3, wherein the woven mesh, knitted fabric or foil comprises metal or inorganic material.

5. A process as claimed in claim 4, wherein the woven mesh, knitted fabric or foil comprising metal is, prior to vapor deposition and/or sputtering, heated at from 400 to 1100°C in an oxygen-containing atmosphere for from 0.5 to 24 hours.

6. A process as claimed in any of claims 1 to 5, wherein the substances active as catalyst are selected from among the elements of transition groups I and/or VII and/or VIII, and/or the promoters are selected from among the elements of main groups III, IV, V, and VI and transition groups II, III, VI and VII of the Periodic Table of the Elements.

7. A process as claimed in any of claims 1 to 6, wherein the hydrogenation of C₂ and/or C₃ streams is carried out in the gas phase.

8. A process as claimed in any of claims 1 to 6, wherein the hydrogenation of C₃, C₄, C₅ and/or C₆ streams is carried out in the liquid phase or in a mixed liquid/gas phase having at least 50% by weight of the fluid in the liquid phase.

9. A process for catalytic distillation in which a hydrogenation as defined in any of the process claims 1 to 6 is combined with a simultaneous distillation or rectification over the catalyst packing, where the fluids may be C₃, C₄, C₅ and/or C₆ streams.

## Revendications

1. Procédé d'hydrogénation sélective d'hydrocarbures polyinsaturés en C₂-C₆, dans des flux de C₂, C₃, C₄, C₅ ou C₆ qui contiennent ceux-ci et proviennent d'un vapocraquage ou d'un craquage catalytique, pour former les hydrocarbures mono-insaturés correspondants, par contact avec une masse de catalyseur en présence d'hydrogène libre, **caractérisé en ce que** la masse de catalyseur peut être préparée par application d'au moins une substance à activité catalytique et éventuellement au moins une substance à activité de promoteur sur du tissu, du tricotage ou des feuilles en tant que matériau de support.

2. Procédé selon la revendication 1, **caractérisé en ce que** l'application de la au moins une substance à activité catalytique et éventuellement d'au moins une substance à activité de promoteur est entreprise par métallisation sous vide et/ou pulvérisation cathodique ou par imprégnation.

3. Procédé selon la revendication 1 ou 2, **caractérisé en ce que** la masse de catalyseur est constituée d'au moins un monolithe qui est formé par le tissu, le tricotage ou la feuille, sous forme d'une bande.

4. Procédé selon l'une des revendications 1 à 3, **caractérisé en ce que** le tissu, le tricotage ou la feuille est en métal ou en un matériau inorganique.

5. Procédé selon la revendication 4, **caractérisé en ce que** le tissu, le tricotage ou la feuille en métal est, avant la vaporisation sous vide et/ou la pulvérisation cathodique, chauffé(e) pendant 0,5 à 24 heures dans une atmosphère oxygénée, à une température de l'ordre de 400 à 1100°C.

6. Procédé selon l'une des revendications 1 à 5, **caractérisé en ce que** les substances à activité catalytique sont choisies parmi les éléments des sous-groupes I et/ou VII et/ou VIII et que les promoteurs sont choisis parmi les éléments des groupes III, IV, V, VI et/ou les sous-groupes II, III, VI, VII du système périodique des éléments.

7. Procédé selon l'une des revendications 1 à 6, **caractérisé en ce que** l'hydrogénation des flux de C₂ et/ou C₃ est entreprise en phase gazeuse.

8. Procédé selon l'une des revendications 1 à 6, **caractérisé en ce que** l'hydrogénation des flux de C₃, C₄, C₅ et/ou C₆ est entreprise en phase liquide ou dans un mélange de phase liquide/gazeuse où au moins 50% en poids du fluide sont en phase liquide.

9. Procédé de distillation catalytique, **caractérisé en ce qu'**une hydrogénation, telle que définie dans l'une des revendications de procédé 1 à 6, peut être combinée à une distillation ou une rectification simultanées sur la masse de catalyseur, les fluides pouvant être des flux de C₃, C₄, C₅ et/ou C₆.
